# EUROPÄISCHE PATENTSCHRIFT

(11) **EP 2 288 285 B1**
(45) Veröffentlichungstag und Bekanntmachung des Hinweises auf die Patenterteilung: **25.03.2015**
(21) Anmeldenummer: 09768962.4
(22) Anmeldetag: 23.06.2009
(51) Int. Cl.: A61B 1/012

(54) **ENDOSKOP MIT SCHAFTROHR UND OPTIK**
ENDOSCOPE HAVING A SHAFT TUBE AND OPTICS
ENDOSCOPE AVEC TIGE ET OPTIQUE

(30) Priorität: 27.06.2008 DE 102008030130
(43) Veröffentlichungstag der Anmeldung: 02.03.2011
(73) Patentinhaber: OLYMPUS WINTER & IBE GMBH, 22045 Hamburg (DE)
(72) Erfinder: REIF, Matthias, 20259 Hamburg (DE); WOSNITZA, Thomas, 21337 Lüneburg (DE)
(74) Vertreter: Hausfeld, Norbert
(86) Internationale Anmeldenummer: PCT/EP2009/004499
(87) Internationale Veröffentlichungsnummer: WO 2009/156113

(56) Entgegenhaltungen:
- EP-A- 0 072 689
- WO-A-2005/037088
- GB-A- 2 406 281
- US-A- 4 146 019
- US-A1- 2006 173 244
- US-A1- 2006 178 560

## Beschreibung

Die Erfindung betrifft ein chirurgisches Endoskop mit einem Schaftrohr und einer darin angeordneten Optik, mit der der Bereich vor dem distalen Ende des Schaftrohres beobachtet werden kann.

Es ist bekannt, Endoskope zur Resektion der Prostata einzusetzen. Sie weisen ein im Schaftrohr angeordnetes Messer auf, das vor dem distalen Ende des Schaftrohres Gewebe abschneiden kann. Üblicherweise ist dieses Messer als HFbeaufschlagte Schneidschlinge ausgebildet. Da bei diesen Arbeiten durch Blutungen schlechte Sichtverhältnisse herrschen, muss gut gespült werden.

Solche zur Resektion verwendeten Endoskope weisen ein Dauerspülsystem auf, bei dem dauernd in den Sichtbereich gespült und aus diesem abgesaugt wird. Eingespült wird dabei durch ein inneres Schaftrohr und abgesaugt wird durch den Ringraum zwischen dem inneren und dem äußeren Schaftrohr, wobei das äußere Schaftrohr im distalen Bereich mit seitlichen Löchern versehen ist, durch die abgesaugt wird.

Die beschriebenen Resektionsgeräte werden bei der klassischen transurethralen Resektion eingesetzt, bei der relativ kleine Gewebestücke abgeschnitten werden, die mit dem Spülwasser herausgesaugt werden können. Dabei muss von Zeit zu Zeit der Einsatz aus dem äußeren Schaftrohr herausgenommen werden, um auch größere Stücke durch den vergrößerten Querschnitt abfließen zu lassen.

Neuerdings setzen sich jedoch Prostata-Resektionsmethoden durch, wie z. B. TUEB (Trans Urethral Enucleation Bipolar) oder HOLEP (HOlmium Laser Enucleation Prostate) bei denen das Gewebe in größere Stücke zerlegt wird, die durch das Schaftrohr des Endoskops nicht mehr herausgeführt werden können. Bei diesen Resektionsarbeiten entstehen also in der Blase liegende große Gewebestücke, die dort zunächst zerkleinert werden müssen, bevor sie durch das Schaftrohr nach außen abgeführt werden können.

Für diese Zerkleinerungszwecke werden Morzellatoren verwendet, die Gewebe zerkleinern und vom Ort der Zerkleinerung absaugen. Die abgesaugte Flüssigkeit muss im Sinne einer Dauerspülung ähnlich wie bei den Prostataresektionsmethoden nachgerührt werden.

Ähnliche Probleme bestehen bei anderen chirurgischen Instrumenten, die absaugen, wie beispielsweise bei einfachen Saugrohren, mit denen zum Beispiel Gewebereste, Steinfragmente oder dergleichen vom Blasenboden abgesaugt werden. Auch hier gibt es wie bei den zuvor beschriebenen Morzellierungsarbeiten die Problemstellung, das Saugrohr bzw. den saugenden Morzellator mit der Saugöffnung gezielt unter optischer Beobachtung manövrieren und dabei die abgesaugte Flüssigkeit nachführen zu müssen.

Ein gattungsgemäßes Endoskop ist aus der US 2008/0091061 A1 bekannt. Bei dieser Konstruktion ist die Optik innerhalb des Saugrohres angeordnet und liegt mit ihrer Saugöffnung in der Stirnfläche des Schaftrohres. Das Verschlusselement wird bei dieser Konstruktion von einem konischen Endstück des Schaftrohres ausgebildet.

Ein Endoskop mit sehr vielfältigen Nutzungsmöglichkeiten ist aus der US 2006/0178560 A1 bekannt.

Die vorliegende Erfindung hat die Aufgabe, das gezielte Manövrieren der Saugöffnung unter optischer Beobachtung zu verbessern.

Diese Aufgabe wird mit den Merkmalen des Anspruches 1 gelöst.

Erfindungsgemäß ist ein Endoskop mit Schaftrohr und darin liegender Optik vorgesehen und mit einem Saugrohr, z.B. in Form eines saugenden Morzellators, dessen Saugöffnung vor dem distalen Ende des Schaftrohres des Endoskops im Beobachtungsfeld der Optik liegt. Saugöffnung und das distale Ende des Schaftrohres sind also voneinander beabstandet. Bei dieser Konstruktion ist der Morzellator bzw. das Saugrohr sehr gut steuerbar und beobachtbar und kann sehr präzise die ausgewählten Stücke ansaugen bzw. morzellieren. Da mit dem Saugrohr bzw. Morzellator angesaugt wird, werden die seitlichen Löcher im Schaftrohr zur Flüssigkeitszufuhr genutzt, um eine Dauerspülung mit saugender Zufuhr und Abfuhr von Flüssigkeit im Arbeitsbereich zu ermöglichen, was zu einem klaren Sichtfeld führt. Dabei versperrt das Verschlusselement das Schaftrohr in axialer Richtung, so dass Flüssigkeit nicht direkt axial aus dem Schaftrohr ausströmen kann, sondern nur seitlich. Es ergibt sich dadurch eine Strömung mit guter Spülwirkung, jedoch ohne Störung durch starke axiale Strömung. Das das Schaftrohr am distalen Ende versperrende Verschlusselement ist am Endbereich der Optik ausgebildet und kann mit dieser eingesetzt werden.

Vorteilhaft gemäß Anspruch 2 sind die Optik und der Morzellator aus dem Schaftrohr herausnehmbar. Das erleichtert die Reinigung zur Wiederverwendung und schafft Kombinationsmöglichkeiten mit anderen Instrumenten.

Vorteilhaft ist dabei nach Anspruch 3 das Schaftrohr derart ausgebildet, dass es auch als Außenschaft eines eingangs erwähnten Resektionsgerätes verwendbar ist. Das schafft den großen Vorteil, dass derselbe Außenschaft für die Resektion und auch für das anschließende Morzellieren verwendbar ist. Er kann also während der gesamten Operation in der Urethra liegen bleiben, so dass die Beeinträchtigungen des Patienten durch mehrfaches Einführen und Herausziehen des Schaftes verringert werden.

In der Zeichnung ist die Erfindung beispielsweise und schematisch dargestellt. Es zeigen:
- Fig. 1: einen Achsschnitt durch den distalen Endbereich des Schaftrohres eines erfindungsgemäßen Endoskops,
- Fig. 2: eine Seitenansicht des Schaftrohres,
- Fig. 3: eine Seitenansicht eines Einsatzes für das Schaftrohr der Figur 2,
- Fig. 4: einen Schnitt nach Linie 4 - 4 in Figur 1 und
- Fig. 5: einen Schnitt nach Linie 5 - 5 in Figur 1

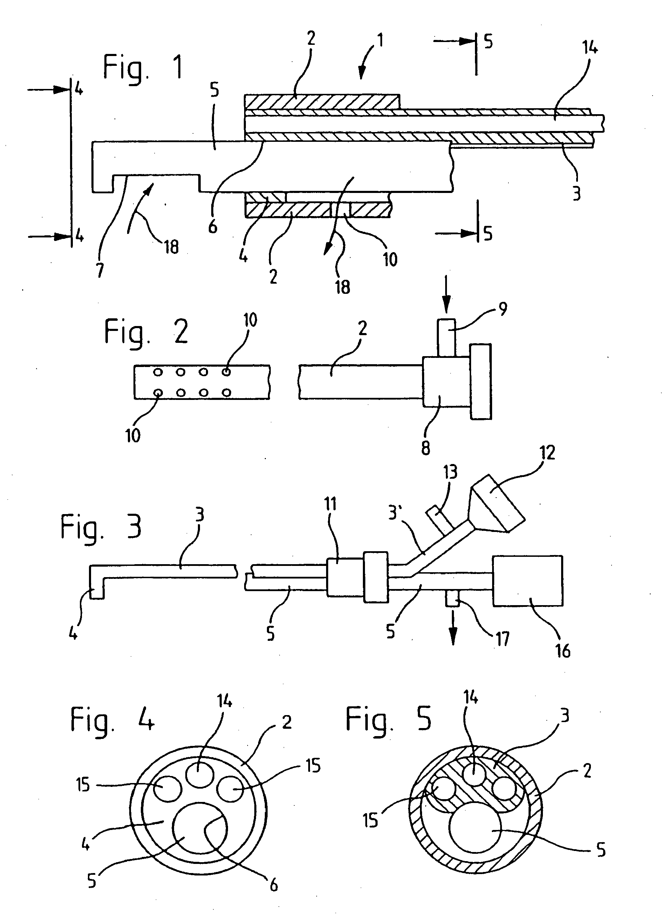

Fig. 1 zeigt im Längsschnitt den distalen Spitzenbereich eines urologischen Endoskops 1 mit einem die Außenwand bildenden Schaftrohr 2 und einer darin angeordneten Optik 3, die wie die Figuren 1 und 3 zeigen, im distalen Endbereich als Verschlusselement 4 ausgebildet ist, das den Innenquerschnitt des Schaftrohres 2 verschließt. Proximal anschließend an das Verschlusselement 4 ist jedoch, wie die Fig. 1 an der Schnittlinie 5 - 5 zeigt, der Querschnitt der Optik 3 kleiner, was insbesondere auch die Fig. 5 deutlich zeigt.

In dem neben der Optik 3 an dieser Stelle verbleibenden freien Innenquerschnitt des Schaftrohres 2 ist Platz, um einen langgestreckten, stabförmigen Morzellator 5 einzuführen, der, wie die Figuren 1 und 4 zeigen, eine parallel zur Achse des Schaftrohres 2 angeordnete Bohrung 6 im Verschlusselement 4 durchläuft und mit seinem distalen Endbereich vor dem distalen Ende des Schaftrohres 2 angeordnet ist. Dort weist der Morzellator 5 eine seitliche Saugöffnung 7 auf, die zum morzellieren und ansaugen des morzellierten Gutes dient.

Fig. 2 zeigt das Schaftrohr 2 in Seitenansicht. Am proximalen Endbereich 8 des Schaftrohres 2 setzt ein Spülwasserzulaufstutzen 9 an, mit dem in Pfeilrichtung Flüssigkeit in das Innere des Schaftrohres 2 eingeführt werden kann. Im distalen Endbereich des Schaftrohres 2 weist dieses nach allen Seiten Löcher 10 auf, von denen eines auch in Fig. 1 zu sehen ist.

Wie Fig. 3 zeigt, weist die Optik 3 in ihrem proximalen Endbereich ein Kupplungsstück 11 auf, das in dem proximalen Endbereich 8 des Schaftrohres 2 eingesteckt und abgedichtet kuppelbar ist. Von dem Kupplungsstück 11 setzt sich die Optik proximal in einem abgewinkelten Stück 3' fort, an dessen Ende ein Okular 12 und ein seitlicher Lichtleiteranschlussstutzen 13 angeordnet sind. Das Okular 12 sitzt am proximalen Ende eines Bildleiters 14, der die Optik 3 auf ihrer Länge durchläuft. Der Lichtleiteranschlussstutzen 13 sitzt am proximalen Ende zweier paralleler Lichtleiter 15, die ebenfalls die Länge der Optik 3 durchlaufen und in deren distaler Stirnfläche abstrahlen.

Anders als im Ausführungsbeispiel erläutert, kann die Optik 3 zum Beispiel als Videooptik ausgebildet sein, bei der im distalen Endbereich eine Videokamera angeordnet ist, von der ein den Bildleiter bildendes Videokabel in proximaler Richtung verläuft. In der Optik können die Lichtleiter fehlen, die z. B. getrennt vom dargestellten Endoskop angeordnet sein können.

Wie Fig. 3 zeigt, durchläuft der Morzellator 5 abgedichtet das Kupplungsstück 11 der Optik 3 und steht aus diesem proximal heraus. An seinem proximalen Ende ist ein Morzellatorantrieb 16 angeordnet, sowie ein seitlicher Stutzen 17, aus dem Flüssigkeit aus dem flüssigkeitsdurchgängigen Inneren des Morzellators 5 abgesaugt wird.

Abweichend von der dargestellten Konstruktion kann anstelle des Morzellators 5 ein einfaches Saugrohr verwendet werden, dass beispielsweise wie der dargestellte Morzellator 5 gestaltet sein kann, nur ohne Morzellierfunktion. Auch andere saugende Instrumente können anstelle des Morzellators 5 verwendet werden, wie z. B. arthroskopische Shaver.

Wird der Morzellator 5, der durch Herausziehen in proximaler Richtung von der Optik 3 trennbar ist, in diese gemäß Konfiguration der Fig. 3 eingesetzt und wird die Optik 3 wiederum in das Schaftrohr 2 der Fig. 2 eingesetzt, so ergibt sich im distalen Endbereich die in Fig. 1, 4 und 5 dargestellte Konfiguration. Dabei ist das Innere des Schaftrohres 2 an seinem distalen Ende durch den Endkörper 4 abgedichtet. Durch den Spülwasserzulaufstutzen 9 in das Schaftrohr 2 eingeführte Spülflüssigkeit tritt durch die seitlichen Löcher 10 aus dem Schaftrohr 2 aus und wird vom Morzellator 5 durch die Öffnung 7 angesaugt, durch die von außen auch das rotierende Messer des Morzellators, das vom Antrieb 16 angetrieben ist, zugänglich ist. Der Flüssigkeitszu- und -ablauf ist in Fig. 1 mit Pfeilen 18 angedeutet.

Wie bereits erläutert, lassen sich die Optik 3 und der Morzellator 5 in proximaler Richtung aus dem Schaftrohr 2 herausziehen. Dabei ist, wie die schematische Darstellung der Fig. 2 erkennen lässt, der Außenschaft 2 in seinem proximalen Endbereich 8 derart ausgebildet, dass an ihm nicht nur die Optik 3 mit ihrem Kupplungsstück 11 ankuppelbar ist, sondern dass am Schaftrohr 2 alternativ auch die Resektionseinsätze von Resektionsgeräten passend ansetzbar sind, die z. B. nach den Methoden TUEB oder HOLEP arbeiten.

Wird das Schaftrohr 2 als Außenschaft eines Resektionsgerätes verwendet, so wird üblicherweise in axialer Richtung aus dem distalen Ende des Schaftes heraus in distaler Richtung Flüssigkeit zugeführt und durch die seitlichen Löcher im Außenschaft abgesaugt. Bei der vorliegenden Verwendung des Schaftrohres 2 zusammen mit einem Saugrohr bzw. einem saugenden Morzellator 5, wird vom Saugrohr bzw. Morzellator abgesaugt und durch die Löcher 10 Flüssigkeit zugeführt. Dabei kann unter Umständen schon ein einziges Loch 10 ausreichen.

Wie mit den Pfeilen 18 in Fig. 1 dargestellt soll dabei zwischen Flüssigkeitszufuhr und Flüssigkeitsabsaugung eine Kreisströmung erreicht werden. Auf jeden Fall soll aber durch das Verschlusselement 4 verhindert werden, dass der Flüssigkeitszustrom axial erfolgt. Das könnte zu morzellierende Gewebestücke wegschwemmen und somit das Morzellieren oder Absaugen verhindern, während bei der erfindungsgemäßen Konstruktion gemäß Fig. 1 die Flüssigkeitsführung Gewebeteilchen in Richtung auf die Saugöffnung 7 zutreibt.

## Patentansprüche

1. Chirurgisches Endoskop (1) mit einem Schaftrohr (2), das in seinem proximalen Endbereich (8) an einen Spülwasserzulauf (9) anschließbar ist und im distalen Endbereich wenigstens ein seitliches Loch (10) aufweist, und mit einer das Schaftrohr (2) durchlaufenden Optik (3), die mit kleinerem Querschnitt als der Innenquerschnitt des Schaftrohres (2) ausgebildet ist und über ihre Länge von einem Bildleiter (14) durchlaufen ist, wobei im freibleibenden Innenquerschnitt des Schaftrohres (2) ein Saugrohr (5) angeordnet ist, das proximal an eine Saugeinrichtung (17) angeschlossen ist, und wobei im distalen Endbereich des Schaftrohres (2) ein dessen restlichen freien Innenquerschnitt verschließendes Verschlusselement (4) angeordnet ist, **dadurch gekennzeichnet, dass** das Saugrohr (5) neben der Optik (3) und mit seiner Saugöffnung (7) distal vor dem Schaftrohr (2) angeordnet ist und dass der distale Endbereich der Optik (3) das Verschlusselement (4) ausbildet.

2. Endoskop nach Anspruch 1, **dadurch gekennzeichnet, dass** die Optik (3) und das Saugrohr (5) aus dem Schaftrohr (2) herausnehmbar ausgebildet sind.

3. Endoskop nach Anspruch 2, **dadurch gekennzeichnet, dass** das Schaftrohr (2) als Außenschaft eines Resektionsgerätes für die transurethrale Prostataresektion ausgebildet ist.

## Claims

1. A surgical endoscope (1) having a shaft tube (2) that can be connected to a flushing water supply unit (9) in its proximal end region (8) and comprises at least one lateral hole (10) in its distal end region, and having an optical system (3) that passes through the shaft tube (2) and is formed with a cross-section that is smaller than the internal cross-section of the shaft tube (2), with an image guide (14) running through said optical system over the latter's entire length, wherein a suction tube (5) is arranged in the open internal cross-section of the shaft tube (2), said suction tube (5) being connected to a suction device (17) at its proximal end, and wherein a closing element (4) closing the remaining open internal cross-section of the shaft tube (2) is arranged in the latter's distal end region,
**characterised in that**
- the suction tube (5) is arranged next to the optical system (3) and, with its intake opening (7), distal from the shaft tube (2) and that the distal end region of the optical system (3) forms the closing element (4).

2. The endoscope according to Claim 1, **characterised in that** the optical system (3) and the suction tube (5) are formed such that they can be removed from the shaft tube (2).

3. The endoscope according to Claim 2, **characterised in that** the shaft tube (2) is formed as an outside shaft of a resection device for transurethral resection of the prostate.

## Revendications

1. Endoscope chirurgical (1) avec une gaine (2) qui, au niveau de sa section d'extrémité proximale (8), peut être branchée sur une alimentation en eau de rinçage (9) et présente, dans sa section d'extrémité distale, au moins un orifice latéral (10), et avec une optique (3) traversant la gaine (2), cette optique ayant une section plus petite que la section intérieure de la gaine (2) et étant traversée sur toute sa longueur par un conducteur optique (14), un tube d'aspiration (5) branché du côté proximal sur un dispositif d'aspiration (17) étant agencé dans la partie libre de la section intérieure de la gaine (2), un élément obturateur (4) étant agencé dans la section d'extrémité distale de la gaine (2) dont il obture le reste de la section intérieure libre, **caractérisé en ce que** le tube d'aspiration (5) est agencé à côté de l'optique (3), son ouverture d'aspiration (7) étant placée du côté distal devant la gaine (2), et **en ce que** la section d'extrémité distale de l'optique (3) forme l'élément obturateur (4).

2. Endoscope selon la revendication 1, **caractérisé en ce que** l'optique (3) et le tube d'aspiration (5) sont conformés de façon a pouvoir être extraits de la gaine (2).

3. Endoscope selon la revendication 2, **caractérisé en ce que** la gaine (2) est réalisée sous forme de gaine externe d'un résecteur pour la résection transurétrale de la prostate.
